**Europäisches Patentamt**

**European Patent Office**

**Office européen des brevets**

(11) Veröffentlichungsnummer: **0 325 297**

**A1**

(12) # EUROPÄISCHE PATENTANMELDUNG

(21) Anmeldenummer: 89101088.6

(51) Int. Cl.⁴: **A61F 2/16**

(22) Anmeldetag: 23.01.89

(30) Priorität: 22.01.88 SU 4362316

(43) Veröffentlichungstag der Anmeldung:
26.07.89 Patentblatt 89/30

(84) Benannte Vertragsstaaten:
**DE ES FR GB IT NL**

(71) Anmelder: **MEZHOTRASLEVOI NAUCHNOTEKHNICHESKY KOMPLEX "MIKROKHIRURGIA GLAZA"**
**Beskudnikovsky bulvar, 59a**
**Moskau(SU)**

(72) Erfinder: **Fedorov, Svyatoslav Nikolaevich**
**Ulitsa Dostoevskogo, 12, kv.32**
**Moscow(SU)**
Erfinder: **Ivanov, Igor Leonidovich**
**Ulitsa Avtozavodskaya, 19 Korpus 1,kv.7**
**Moscow(SU)**
Erfinder: **Pashinova, Nadezhda Fedorovna**
**Ulitsa Koshtoyantsa, 39, kv.60**
**Moscow(SU)**
Erfinder: **Zakharov, Dmitry Valerievich**
**Ulitsa Dubninskaya, 71, kv.109**
**Moscow(SU)**

(74) Vertreter: **Patentanwälte Beetz sen. - Beetz jun. Timpe - Siegfried - Schmitt-Fumian-Mayr**
**Steinsdorfstrasse 10**
**D-8000 München 22(DE)**

(54) **Intraokularlinse.**

(57) Die Erfindung betrifft eine künstliche Augenlinse (1), die zur Implantation in den Linsenkapselsack bestimmt ist und eine Linse (2) und eine am Außenrand der Linse (2) vorgesehene Abstützung in Form einer einzigen, ovalen Schleife (3) aufweist, die in zwei Punkten (5, 6) mit der Seitenfläche (4) der Linse (2) verbunden ist, zumindest die Hälfte des Umfangs der Linse (2) umfaßt und in einer zu einer Umfangsebene parallelen Ebene liegt. Die große und die kleinere Achse der Schleife (3) liegen vorteilhaft in zwei zueinander senkrechten Ebenen der Linse (2), wobei das Verhältnis der Längen der großen und der kleineren Achse der Schleife (3) 1,3 bis 1,4 beträgt.

Die erfindungsgemäße künstliche Augenlinse kann bei der chirurgischen Behandlung des Kataraktes beliebiger Ätiologie vorteilhaft eingesetzt werden.

FIG. 1

## Intraokularlinse

Die vorliegende Erfindung bezieht sich auf eine Intraokularlinse, d.h. eine künstliche Augenlinse, die zur Verwendung bei der chirurgischen Behandlung des Kataraktes beliebiger Ätiologie bestimmt ist.

In den letzten Jahrzehnten gewannen künstliche intraokulare Augenlinsen immer weitgehendere Verbreitung, die bei der chirurgischen Behandlung des Kataraktes nach Entfernen der mit Star behafteten natürlichen Augenlinse in das Auge eingesetzt werden.

Bekannt sind sehr verschiedene Arten künstlicher Augenlinsen, die sich hauptsächlich durch die konstruktive Ausführung der Abstützungselemente voneinander unterscheiden, durch die eine Festlegung der Linse im Auge erzielt wird. Die Form und die Anordnung dieser Abstützungen legen ihrerseits die Anordnung der künstlichen Augenlinse fest. Dementsprechend werden die bekannten Augenlinsen je nach ihrer örtlichen Positionierung übereinkunftsgemäß in Vorderkammer-, Sehloch- und Hinterkammerlinsen eingeteilt.

Die ersten beiden Arten der künstlichen Augenlinsen sind mit einigen Nachteilen behaftet, von denen die Hauptnachteile in der Reaktivität des Auges wegen des Drucks der Abstützungselemente bzw. der sogenannten haptischen Tastelemente auf die Gewebe und Gefäße in der Umgebung und in der daraus resultierenden Irritation und Beeinträchtigung sowie darin bestehen, daß bisweilen ein gesundheitsschädlicher Augenzustand auftritt. Hierdurch sowie auch durch einen ständigen Druck auf die Hinterkapsel, den Ziliarkörper und die Ziliarfortsätze können dystrophische Veränderungen in diesen Bereichen auftreten. Darüber hinaus führt der ununterbrochene Kontakt mit dem rückwirkenden Irispigmentepithel wiederum zu einer dystrophischen Veränderung im Irispigmentblatt.

Aus diesen Gründen haben die künstlichen Hinterkammeraugenlinsen größere Verbreitung gefunden, wobei die Augenlinse zur Anordnung im Linsenkapselsack vorgesehen wird, da in diesem Fall die Abstützung der Augenlinse mit einem inaktiven Gewebe ohne Gefäße und Nervenenden in Wechselwirkung tritt und somit keine Rückwirkung auf das Auge eintritt. In der Praxis tritt jedoch das Problem auf, daß der Chirurg bei der Fixierung der Linse in der Ziliarfurche nicht immer sicher ist, ob die Linse bei dieser Operation auch genau positioniert ist, weil bei der Anordnung der Linse in der Ziliarfurche keinerlei visuelle Kontrolle möglich ist.

Deshalb besteht in der Praxis die Tendenz, in der Kapsel der natürlichen Augenlinse einzusetzende künstliche Linsen zu verwenden.

Üblicherweise bestehen derartige künstliche Augenlinsen aus dem eigentlichen Linsenteil und einer Abstützung, die aus einem federnden Werkstoff hergestellt und außerhalb der Linse in Form von zwei einander diametral gegenüberliegenden Schleifen angeordnet ist, die in der optischen Ebene der Linse liegen (s. z.B. SU-A-1 134 196). Auch diese künstlichen Augenlinsen sind jedoch mit einigen Nachteilen behaftet. So besteht beispielsweise keine zuverlässige Garantie dafür, daß die beiden Abstützungselemente in den Kapselsack gelangen, während die Festlegung nur an einem Abstützungselement in Anbetracht dieser konstruktiven Ausführung der Abstützung wegen des Inkontaktkommens mit den reagierenden Geweben unsicher und unerwünscht ist. Ein wiederholter Versuch, das andere Abstützungselement hineinzuführen, ruft andererseits nur eine weitere Verletzung des Auges des Patienten hervor. Das Einsetzen der künstlichen Augenlinse der beschriebenen Art ist mit einer zusätzlichen traumatischen Einwirkung verbunden, da hierbei die Regenbogenhaut gehoben werden muß, wodurch die Operationsdauer verlängert wird und die Bedingungen für die nachfolgende Rehabilitation verschlechtert werden.

Der vorliegenden Erfindung liegt die Aufgabe zugrunde, eine künstliche Augenlinse mit einer Abstützung anzugeben, bei der sichergestellt ist, daß die künstliche Augenlinse samt der Abstützung innerhalb des Kapselsacks eingesetzt und die Augenlinse sicher festgelegt werden kann.

Die Aufgabe wird anspruchsgemäß gelöst. Die abhängigen Ansprüche betreffen günstige Ausführungsformen.

Die erfindungsgemäße künstliche Augenlinse, die zur Implantation in den Linsenkapselsack vorgesehen ist, umfaßt eine Linse und eine am Außenrand der Linse angeordnete, federnde Abstützung; sie ist dadurch gekennzeichnet, daß die Abstützung eine einzige, ovale Schleife darstellt, die innerhalb des Bereichs der Randdicke der Linse in einer zu einer Umfangsebene parallelen Ebene liegt, in zwei voneinander beabstandeten Punkten mit der Seitenfläche der Linse verbunden ist und die Linse in einem Umfangsbereich umfaßt, der einem Zentriwinkel von mindestens 180° entspricht, wobei die große und die kleinere Achse der ovalen Schleife in zueinander senkrechten Diametralebenen der Linse liegen.

Die Länge der großen Achse der ovalen Schleife stimmt dabei günstigerweise mit dem Durchmesser des Linsenkapselsacks des Auges überein, für das die Augenlinse vorgesehen ist.

Das Verhältnis der Länge der großen Achse zur Länge der kleineren Achse der ovalen Schleife beträgt vorteilhaft etwa 1,3:1 bis etwa 1,4:1.

Besonders vorteilhafte Ausführungsformen sind durch folgende Merkmale gekennzeichnet, die unabhängig voneinander vorliegen können:

- Die ovale Schleife liegt in der Umfangsebene, die in der Mitte der Seitenfläche der Linse verläuft.

- Die ovale Schleife umfaßt die Linse in einem Umfangsbereich, der einem Zentriwinkel $\alpha$ von etwa 300° entspricht.

- Die ovale Schleife ist in Bezug auf die durch ihre kleinere Achse verlaufende Diametralebene symmetrisch.

- Der Querschnitt der ovalen Schleife weist in einem beliebigen Abschnitt oder über ihre gesamte Länge die Form eines Vierecks mit abgerundeten Ecken und einem Seitenverhältnis von etwa 1:1 bis etwa 3:1 auf.

- Die Enden der ovalen Schleife sind in im Bereich der Seitenfläche vorgesehene Bohrungen eingepreßt, oder die Linse und die ovale Schleife sind zusammen als Ganzes einstückig hergestellt.

- Die Enden der ovalen Schleife gehen unter einem zur Schleife hin gemessenen Winkel zur entsprechenden Tangente in den Verbindungspunkten in der Ebene der ovalen Schleife in die Linse über, der kleiner als 90° ist.

- Die Enden der ovalen Schleife gehen unter einem zur Schleife hin gemessenen Winkel zur entsprechenden Tangente in den Verbindungspunkten in der Ebene der ovalen Schleife in die Linse über, der etwa gleich 180° minus dem halben Zentriwinkel ist, d.h., die beiden Enden der ovalen Schleife verlaufen etwa richtungsgleich.

- Die Linse und/oder die ovale Schleife bestehen aus einem Kunststoff, insbesondere aus Poly(methylmethacrylat).

- Die Linse und/oder die ovale Schleife sind durch Gießen, Blockpolymerisation oder Spritzgießen hergestellt.

Durch diese konstruktive Ausbildung der erfindungsgemäßen Intraokularlinse mit einer einzigen Abstützung in Form einer ovalen Schleife kann diese Augenlinse unter visueller Kontrolle einfach, bequem und vollständig in einem Arbeitsgang in den Linsenkapselsack eingeführt und durch geeignete Wahl der Abmessungen der ovalen Schleife unter Berücksichtigung der Größe des Linsenkapselsacks sowie des Längenverhältnisses zwischen der großen und der kleineren Achse und durch die Orientierung der Schleife in Bezug auf die Linse sicher festgelegt werden.

Ein weiterer Vorteil der Erfindungskonzeption besteht darin, daß bei der Implantation der erfindungsgemäßen Augenlinse keine Anhebung der Regenbogenhaut mehr erforderlich ist, wodurch ein traumatisches Verletzungsrisiko bei der Operation praktisch entfällt und die Bedingungen für die postoperative Rehabilitation verbessert werden.

Das Längenverhältnis der großen und der kleineren Achse ist von wichtiger Bedeutung für die Erzielung der höchsten Lagestabilität der Augenlinse innerhalb des Linsenkapselsacks bei minimalem Druck auf die Kapselwandung. Wird der Wertebereich des oben definierten Längenverhältnisses in relevantem Maße über- bzw. unterschritten, so können die genannten Bedingungen je nach dem konkreten Bau des Auges des Patienten nicht eingehalten werden. Ist das Längenverhältnis geringer als 1,3, kann eine Dezentrierung der künstlichen Augenlinse auftreten, während bei einem Verhältnis von über 1,4 ein zu hoher Druck auf die Wandung des Linsenkapselsacks auftreten kann.

Die Ausbildung des Querschnitts der ovalen Schleife in Form eines Vierecks mit abgerundeten Ecken und einem Seitenverhältnis im Bereich von etwa 1:1 bis etwa 3:1 ist besonders vorteilhaft, da hierdurch die Abstützung der Augenlinse beim Einsetzen nicht von der Umfangsebene der Linse abweicht.

Im folgenden wird die Erfindung anhand eines konkreten Ausführungsbeispiels und der Zeichnungen näher erläutert; es zeigen:

Fig. 1: Eine erfindungsgemäße künstliche Augenlinse in der Draufsicht;

Fig. 2: eine Seitenansicht der Augenlinse von Fig. 1:

Fig. 3: einen Querschnitt durch die ovale Schleife der Augenlinse von Fig. 1 längs der Linie III-III in Fig. 1;

Fig. 4: eine erfindungsgemäße Augenlinse, die mit einer schematisch gezeigten Pinzette erfaßt wird, bei der Einführung in das Auge, sowie

Fig. 5: eine schematische Darstellung eines Auges mit darin eingesetzter künstlicher Augenlinse gemäß der Erfindung.

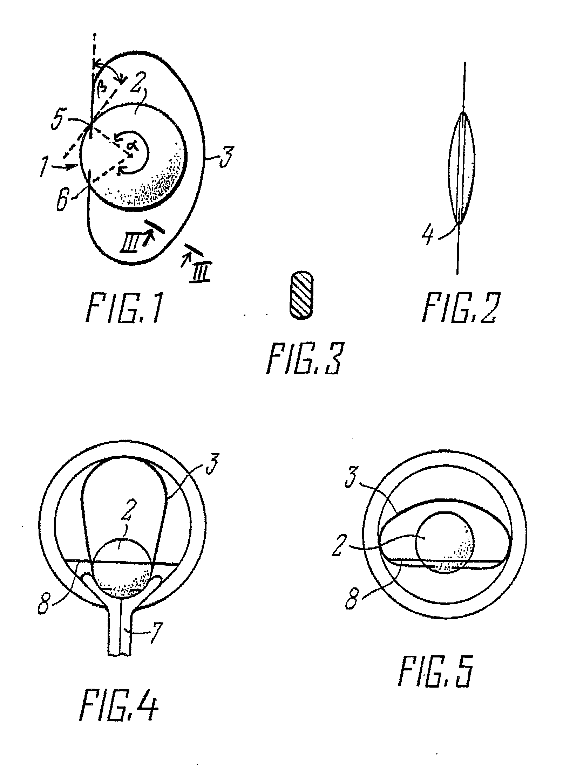

Wie aus den Figuren 1 und 2 ersichtlich ist, besteht die künstliche Augenlinse 1 aus einer Linse 2, die den optischen Teil darstellt, und einer einzigen, ovalen Schleife 3 als Abstützung.

Die Linse 2 kann aus Poly(methylmethacrylat), aber auch aus einem anderen, bekannten Werkstoff hergestellt werden, der gegenüber dem fließenden Medium des Auges und den umgebenden Geweben biologisch inert ist.

Die als einzige, ovale bzw. längliche Schleife 3 ausgeführte Abstützung ist an der Seitenfläche 4 der Linse 2 in den Punkten 5 und 6 befestigt.

Als Materialien zur Herstellung einer solchen Schleife kommen beliebige federnde Werkstoffe in Frage, die gegenüber dem flüssigen Medium des Auges und den umgebenden Geweben biologisch inert sind. Die Befestigung erfolgte beim vorliegenden Ausführungsbeispiel durch Ausbohren von Bohrungen in der Seitenfläche 4 der Linse 2, Einsetzen der Enden der Schleife 3 in diese Bohrungen und Einpressen darin. Diese Befestigung muß so vorgenommen werden, daß sich die Schleife 3 außerhalb der Linse 2 in einer um einer Umfangsebene parallelen Ebene im Bereich der Seitenfläche 4 befindet, wie aus Fig. 1 und 2 zu ersehen ist, wobei die Schleife 3 die Linse 2 innerhalb eines Umfangsbereichs umfaßt, der einem Zentriwinkel $\alpha$ von etwa 300° entspricht. Selbstverständlich kann der Umfassungsbereich auch kleiner oder größer sein, in jedem Falle aber darf dieser Umfassungsbereich nicht kleiner als der halbe Umfang der Linse sein, wobei die Befestigungspunkte 5, 6 der Schleife 3 an der Seitenfläche 4 voneinander hinreichend beabstandet sein müssen, da sich anderenfalls die Augenlinse nach dem Einsetzen in das Auge in einem unstabilen Zustand befinden kann.

Der zwischen den Enden der Schleife 3 und der zugehörigen Tangente in den Punkten 5 bzw. 6 zur Schleife 3 hin gemessene Winkel $\beta$ ist vorzugsweise kleiner als 90°. In einem vorteilhaften Spezialfall, bei dem die Enden der Schleife 3 richtungsgleich sind, ist der Winkel $\beta \approx 180 - \alpha/2$.

Die ovale Schleife 3 weist eine große, in einer Diametralebene der Linse 2 liegende Achse und eine kleinere, in einer anderen, zur erwähnten Diametralebene senkrechten Diametralebene liegende Achse auf, wobei die beiderseits der Ebene der kleineren Achse einander gegenüberliegenden Schleifabschnitte zueinander symmetrisch sind, diese Ebene also eine Symmetrieebene darstellt. Die große Achse der Schleife 3 wird gleich dem Durchmesser des Linsenkapselsacks ausgewählt, dessen mittleres Maß dem auf diesem Gebiet tätigen Durchschnittsfachmann gut bekannt ist; es beträgt üblicherweise 10 bis 12 mm. Im vorliegenden Ausführungsbeispiel ist das Verhältnis von großer und zu kleinerer Achse gleich 1,35 gewählt. Es kann jedoch auch andere Werte besitzen, die innerhalb des Bereichs von 1,3 bis 1,4 liegen; bei einer Abweichung vom genannten Be reich können die Bedingungen für die Lagestabilität und Zentrierung verschlechtert werden oder der Druck auf die Innenwandung des Linsenkapselsacks stark zunehmen.

Wie aus Fig. 3 zu erkennen ist, weist die Schleife 3 in ihrem Querschnitt die Form eines Rechtecks mit abgerundeten Ecken auf, um so eine Verletzung des Linsenkapselsacks zu vermeiden. Dabei ist das Verhältnis der größeren zu der kleineren Seite des Rechtecks gleich 2:1 gewählt; es kann jedoch auch andere Werte annehmen, und zwar, wie die Praxis bestätigt, im Bereich von etwa 1:1 und bis etwa 3:1, da sonst die Schleife 3 der Augenlinse von der Umfangsebene der Linse abweichen kann.

Die Implantation der erfindungsgemäßen künstlichen Augenlinse wird wie folgt vorgenommen.

Noch vor der Operation wird die größtmögliche Dosis einer 2,0- bis 4 %-igen Novocainlösung durch Injektion verabreicht und Akinesie mit einer Novocainlösung in einer Menge von 2 bis 5 ml hervorgerufen. Hiernach wird eine Ligaturnaht am oberen geraden Muskel angelegt und die vordere Kapsel in ihrem Oberteil mit einer Vannasschere oder einem Diamantmesser eröffnet; die Öffnung 8 ist in Fig. 4 und 5 schematisch dargestellt.

Mit Hilfe eines Sato-Messers wird der Kern der Linse durch Drücken des Skleralteils des Einschnitts entfernt. Es folgen Aspiration und Auswaschung der restlichen Linsenmassen aus dem Kapselsack. Um die erfindungsgemäße künstliche Augenlinse in den so vorbereiteten Kapselsack zu implantieren, wird die als Abstützung dienende Schleife 3 mit einer Pinzette 7 an die Seitenfläche 4 der Linse 3 angedrückt, wie aus Fig. 4 ersichtlich ist. Die mit der Pinzette 7 in diesem Zustand gehaltene Augenlinse wird dann in den Kapselsack eingeführt, wonach die Pinzette 7 geöffnet und aus der Vorderkammer herausgenommen wird. Durch die Federkraft der Schleife 3 nimmt die Abstützung wieder ihre ursprüngliche Gestalt an, wobei die große Achse eine waagerechte Lage einnimmt. Auf diese Weise wird die Linse 2 durch die Abstützung innerhalb des Kapselsacks festgelegt und dorthin zentriert. Hiernach wird das Sehloch mit Acetylcholin verengt. Über der Linse wird nun in der Mitte der Vorderkapsel ein Loch gebildet. Mit der Vannasschere wird dann eine fortlaufende Doppelnaht (cornealis) mit einem Tauchknoten gegen 12 Uhr-Position angelegt. Unter der Konjuktiva werden die Dexasonlösung in einer Menge von 0,4 ml und Gentamycinlösung eingeführt.

Wie aus der Beschreibung der Implantationsoperation folgt, bietet die erfindungsgemäße künstliche Augenlinse die Möglichkeit, Verletzungen der Hornhaut im Laufe der Operation zu vermeiden, da hierbei eine endokapsuläre Implantation erfolgt und die Hornhaut mit dem Blättchen der Vorderkapsel geschützt ist. Dabei wird auch die Gefahr ausgeschlossen, die Hinterkapsel zu zerreißen; außerdem entfällt die Notwendigkeit, die Regenbogenhaut anzuheben oder an das Oberteil der Vorderkapsel heranzuziehen. Die Implantation der erfindungsgemäßen künstlichen Augenlinse ermöglicht ferner die Durchführung der Operation unter Anwendung eines nur sehr kleinen Operationseinschnitts, wodurch ebenfalls die Verletzung vermindert und die Operationsdauer verkürzt werden.

Unter Verwendung der erfindungsgemäßen künstlichen Augenlinsen wurden einige Implantationen durchgeführt, deren Ergebnisse nachstehend erläutert sind.

## Beispiel 1

Patient: 60 Jahre alt. Diagnose: OS - Cataracta complicata. OD - Primärstar. Bei der Einlieferung Visus OD = 0,4 + 1,0 D = 0,6; OS = 0,05 (nicht korrigierbar).

Es wurde eine extrakapsuläre Extraktion des Kataraktes durchgeführt und eine erfindungsgemäße Augenlinse eingesetzt, bei der das Verhältnis der Längen der großen und der kleineren Achse 1,3 und das Seitenverhältnis des viereckigen Querschnitts der Schleife 1,0 betrugen. Die Operations- und Nachoperationsperiode verliefen ohne Komplikationen. Bei Entlassung Visus OD = -0,4 + 1,0 D = 0,6; OS = 0,7. Nach 3 Monaten OD = 0,4 + 1,0 D = 0,6; OS = 1,0.

**Beispiel 2**

Patient: 45 Jahre alt. Diagnose: OD - unreifer Star, OS -Phakosklerose. Bei der Einlieferung Visus OD = 0,3 Sphäre + 1,0 D = 0,8; OS = 0,7 - 0,8 Sphäre + 1,0 D = 1,0.

Es wurde eine extrakapsuläre Extraktion des Kataraktes mit anschließendem Einsetzen einer erfindungsgemäßen Augenlinse durchgeführt, bei der das Verhältnis der Längen der großen und der kleineren Achse 1,36 und das Seitenverhältnis des rechteckigen Querschnitts der Schleife 2:1 betrugen.

Bei Entlassung Visus OD = 0,6 mit Korrektion; OS = 0,7 Sphäre + 1,0 D = 1,0.

Nach 3 Monaten OD = 0,9 bis 1,0; OS = 0,7 Sphäre + 1,0 D = 1,0.

Es wurden ferner auch Implantationen erfindungsgemäßer Augenlinsen mit einem Verhältnis der Längen der großen und der kleineren Achse der Schleife von 1,4:1 und einem Seitenverhältnis des Rechteckquerschnitts von 3:1 vorgenommen, die ebenfalls zu positiven Ergebnissen führten.

Bei der obigen Erläuterung wurden bevorzugte Ausführungsformen der erfindungsgemäßen künstlichen Augenlinse beschrieben, die für den Einsatz innerhalb des Linsenkapselsacks bestimmt ist. Die Erfindungskonzeption ist nicht auf die erläuterten Ausführungsbeispiele beschränkt. Statt des Fräsens der Bohrungen in der Seitenfläche der Linse kann beispielsweise auch ein anderes Herstellungsverfahren angewandt werden, bei dem die Linse und die Abstützung als Ganzes geformt bzw. gegossen werden.

Die Implantation der erfindungsgemäßen künstlichen Augenlinse der beschriebenen Ausführung ist nach der extrakapsulären Extraktion des Kataraktes unter der Bedingung möglich, daß die Linsenkapsel unverändert bleibt, die dosiert eröffnet werden kann. Kontraindikation der Implantation ist eine Zerstörung der gesamten Hinterkapsel während des chirurgischen Eingriffs.

**Ansprüche**

1. Künstliche Augenlinse (1) zur Implantation in den Linsenkapselsack, die eine Linse (2) und eine am Außenrand der Linse angeordnete, federnde Abstützung umfaßt,
**dadurch gekennzeichnet,**
daß die Abstützung eine einzige, ovale Schleife (3) darstellt, die innerhalb des Bereichs der Randdicke der Linse (2) in einer zu einer Umfangsebene parallelen Ebene liegt, in zwei voneinander beabstandeten Punkten (5, 6) mit der Seitenfläche (4) der Linse (2) verbunden ist und die Linse (2) in einem Umfangsbereich umfaßt, der einem Zentriwinkel ( $\alpha$ ) von mindestens 180° entspricht, wobei die große und die kleinere Achse der ovalen Schleife (3) in zueinander senkrechten Diametralebenen der Linse (2) liegen (Fig. 1).

2. Augenlinse nach Anspruch 1, dadurch gekennzeichnet, daß die Länge der großen Achse der ovalen Schleife (3) mit dem Durchmesser des Linsenkapselsacks des Auges übereinstimmt, für das die Augenlinse (1) vorgesehen ist.

3. Augenlinse nach Anspruch 1 oder 2, dadurch gekennzeichnet, daß das Verhältnis der Länge der großen Achse zur Länge der kleineren Achse der ovalen Schleife (3) etwa 1,3 bis etwa 1,4 beträgt.

4. Augenlinse nach einem der Ansprüche 1 bis 3, dadurch gekennzeichnet, daß die ovale Schleife (3) in der Umfangsebene liegt, die in der Mitte der Seitenfläche (4) der Linse (2) verläuft (Fig. 2).

5. Augenlinse nach einem der Ansprüche 1 bis 4, dadurch gekennzeichnet, daß die ovale Schleife (3) die Linse (2) in einem Umfangsbereich umfaßt, der einem Zentriwinkel ( $\alpha$ ) von etwa 300° entspricht (Fig. 1).

6. Augenlinse nach einem der Ansprüche 1 bis 5, dadurch gekennzeichnet, daß die ovale Schleife (3) in Bezug auf die durch ihre kleinere Achse verlaufende Diametralebene symmetrisch ist (Fig. 1).

7. Augenlinse nach einem der Ansprüche 1 bis 6, dadurch gekennzeichnet, daß der Querschnitt der ovalen Schleife (3) in einem beliebigen Abschnitt oder über ihre gesamte Länge die Form eines Vierecks mit abgerundeten Ecken und einem Seitenverhältnis von etwa 1:1 bis etwa 3:1 aufweist.

8. Augenlinse nach einem der Ansprüche 1 bis 7, dadurch gekennzeichnet, daß die Enden der ovalen Schleife (3) in den Punkten 5, 6 in im Bereich der Seitenfläche (4) vorgesehene Bohrungen eingepreßt sind.

9. Augenlinse nach einem der Ansprüche 1 bis 7, dadurch gekennzeichnet, daß die Linse (2) und die ovale Schleife (3) zusammen als Ganzes einstückig hergestellt sind.

10. Augenlinse nach einem der Ansprüche 1 bis 9, dadurch gekennzeichnet, daß die Enden der ovalen Schleife (3) unter einem zur Schleife (3) hin gemessenen Winkel ($\beta$) zur entsprechenden Tangente in den Punkten 5 bzw. 6 in der Ebene der ovalen Schleife (3) in die Linse (2) übergehen, der kleiner als 90° ist.

11. Augenlinse nach einem der Ansprüche 1 bis 10, dadurch gekennzeichnet, daß die Enden der ovalen Schleife (3) unter einem zur Schleife (3) hin gemessenen Winkel ($\beta$) zur entsprechenden Tangente in den Punkten 5 bzw. 6 in der Ebene der ovalen Schleife (3) in die Linse (2) übergehen, der etwa gleich 180 - $\alpha/2$ ist.

12. Augenlinse nach einem der Ansprüche 1 bis 11, dadurch gekennzeichnet, daß die Linse (2) und/oder die ovale Schleife (3) aus einem Kunststoff bestehen.

13. Augenlinse nach Anspruch 12, dadurch gekennzeichnet, daß Linse (2) und ovale Schleife (3) blockpolymerisiert, gegossen oder spritzgegossen sind.

14. Augenlinse nach einem der Ansprüche 1 bis 13, dadurch gekennzeichnet, daß die Linse (2) und/oder die ovale Schleife (3) aus Poly-(methylmethacrylat) bestehen.

FIG.1

FIG.3

FIG.2

FIG.4

FIG.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 681 586 (WOODS) <br> * Figur 7; Ansprüche 1-6 * | 1 | A 61 F 2/16 |
| A | | 4-6,8,9 ,12 | |
| A | US-A-4 203 168 (RAININ et al.) <br> * Figur 6 * | 1 | |
| A | US-A-4 354 286 (KRASNOV et al.) <br> * Figur 1 * | 1 | |
| A | US-A-4 280 232 (HUMMEL) <br> * Figur 5 * | 1 | |
| D,A | SU-A-1 134 196 <br> * Figur * | 1 | |

**RECHERCHIERTE SACHGEBIETE (Int. Cl.4)**

A 61 F 2/00

Der vorliegende Recherchenbericht wurde für alle Patentansprüche erstellt

| Recherchenort | Abschlußdatum der Recherche | Prüfer |
|---|---|---|
| BERLIN | 20-03-1989 | KANAL P K |